# EUROPEAN PATENT APPLICATION

(11) **EP 3 858 311 A1**
(43) Date of publication of application: **04.08.2021**
(21) Application number: 19867941.7
(22) Date of filing: 23.04.2019
(51) Int. Cl.: A61F 13/36, A61B 10/02, A61B 17/42, A61B 17/12

(54) **HEMOSTATIC MEMBER FOR CERVIX, AND HEMOSTATIC MEMBER KIT FOR CERVIX**

(30) Priority: 27.09.2018 KR 20180115334; 11.01.2019 KR 20190003988
(71) Applicant: Endovision Co., Ltd., Daegu 41061 (KR)
(72) Inventor: HONG, Dae Gy, Daegu 42674 (KR); JUNG, Min Ho, Daegu 42758 (KR)
(74) Representative: Viering, Jentschura & Partner mbB Patent- und Rechtsanwälte
(86) International application number: PCT/KR2019/004858
(87) International publication number: WO 2020/067619

(57) **Abstract**

A hemostatic member for cervix, and hemostatic member kit for cervix are proposed. The hemostatic member for the cervix that is made of a hemostatic dressing is formed in a flower shape, has a flower core part at the center and petal parts formed around the flower core part and having lower dressing density than the flower core part, and provides a hemostatic member kit for the cervix that includes the hemostatic member, and an applicator keeping the hemostatic member therein and providing the hemostatic member to an affected part.

## Description

### Technical Field

The present invention relates to a hemostatic member for the cervix that is inserted in an affected part to effectively stop bleeding at a hemostasis-requiring portion in a cervical tissue examination or a surgical operation of the cervix.

### Background Art

When a human body is injured, the blood coagulates due to the coagulation mechanism of the blood even through simple measures if the wound is not deep. However, when a wound is deep or a tissue is taken off for a surgical operation or an examination, it is necessary to artificially stop bleeding from the wound in order to prevent profuse bleeding.

In relation to the present invention, there is Korean Patent No. 10-1700107, titled "Chitosan-based hemostatic dressing member and manufacturing method thereof" by the applicant(s) in the related art.

The chitosan-based hemostatic dressing member of the related art is characterized by including a chitosan dressing member 100% made of a chitosan material, having predetermined size and shape, intended to be applied to an injury or an operation part of a human body to facilitate in hemostasis or recovery of the injury or the operation part, and an assistant member coupled to at least one side of the chitosan dressing member to prevent detachment of a fiber and maintain the shape of the dressing member.

The main use of chitosan was limited to the waste water disposal field such as a cohesive agent, a heavy metal adsorbent, and a dye-waste water purifier, and the agricultural field such as a soil conditioner, an insecticide, an antiviral drug for plant, and an agricultural chemical. However, as the advantages and various characteristics of chitosan are discovered, the use range is increasingly expanding to the food and beverage application field, the health and sanitation application filed, the cosmetic application filed, the fiber-related application field, the medicine application filed, etc.

This application of the related art was intended to provide a hemostatic dressing member that can be generally used using the useful functions of chitosan. However, according to the related art, dressing members can be used stop bleeding from the outer part of a human body that is exposed outside, but there is a limitation in hemostasis using common dressing members when operating on or providing medical treatment to organs having specific structures and shapes inside the human body.

The present invention addresses effective hemostasis for an affected part that is caused when a surgical operation or a tissue examination for diagnosis of cervical cancer of women is performed. To this end, there is a need for a hemostatic member that can achieve effective hemostasis for a corresponding affected part unlike gauze that can be generally used in the related art.

For this necessity, the applicant(s) has made an application of a chitosan-based hemostatic member for a cervix that is intended to be inserted and used in an affected part in a uterus tissue examination or operation and is characterized by having a hemostatic chitosan nonwoven fabric formed in a round lump shape (Korean Patent Application No. 10-2017-0127526).

In the related art, when stopping bleeding using a round hemostatic member after taking off a tissue, it is required to improve the shape.

Further, it is required to improve the structure and shape in order to more quickly and smoothly insert the hemostatic member into an affected art.

### Disclosure

### Technical Problem

The present invention has been devised and an objective of the present invention is to provide a hemostatic member having a flower shape for the cervix to effectively stop bleeding for indications of a hemostasis-requiring portion.

Another objective of the present invention is to provide a hemostatic member kit for the cervix that keeps a hemostatic member, which is made of a hemostatic dressing in a flower shape, in an applicator and provides the hemostatic member to a hemostasis-requiring portion using a handle of the applicator.

### Technical Solution

In order to achieve the objectives, a hemostatic member for a cervix is made of a hemostatic dressing in a flower shape and includes a plurality of petal parts, and the petal parts selectively close in a bud shape or open when applied to an affected part.

Only some of the petal parts may open or all the petal parts may open, depending on a shape of an affected part.

The hemostatic member for a cervix may include: flower core part formed at a center; and the petal parts formed around the flower core part, in which the petal parts may have lower dressing density than the flower core part or the flower core part may be larger in height than the petal parts.

A thread is formed at a lower end of the flower core part, and the thread may further include an X-ray response substance.

The dressing may be any one of chitosan, cellulose (cotton, silk, or hemp), recycled cellulose (rayon or artificial silk), alginate, and casein, or is provided by mixing chitosan in any one of cellulose (cotton, silk, or hemp), recycled cellulose (rayon or artificial silk), alginate, and casein.

The dressing may be provided in a nonwoven fabric type mixed with a hemostatic agent or may be provided in a nonwoven fabric type having a single coating layer or multiple coating layers made of a hemostatic agent on a surface thereof.

The hemostatic agent may be any one or a combination of two or more of carboxymethyl chitosan (CMCS), carboxymethylcellulose (CMC), chitosan succinate, sodium alginate, hyaluronic acid, collagen, gelatin, chondroitin sulfate, poly-γ-glutamic acid, pullulan, kaolin, zeolite, chondroitin sulfate, calcium chloride, calcium chloride thrombin, fibrinogen, catechol, and polyurethane foam.

The hemostatic member may further include an X-ray response substance therein.

A diameter of the flower core part may be 15 ∼ 25mm and a diameter of the hemostatic member at the petal parts may be 30 ∼ 60mm.

The hemostatic member may be formed by rolling in one direction a rolling member elongated in a longitudinal direction and having cuts formed on a side of the rolling member by the number of petals of the petal parts.

The cuts may be formed such that a height and a width increase in a rolling direction so that sizes of the outer petal parts are relatively large and gaps thereof increase.

A size (diameter) of the hemostatic member may be adjusted by piling up a plurality of rolling members or the size (diameter) and density of the hemostatic member may be adjusted by overlapping a plurality of rolling members with stepped difference in a rolling direction.

The flower core part may be formed in a shape in which a lower portion decreases downward in horizontal cross-sectional area.

A hemostatic member kit for a cervix according to the present invention includes: a hemostatic member made of a dressing in a flower shape, including a plurality of petal parts that selectively close in a bud shape or open when applied to an affected part, and having a thread at a lower end of the flower core part; and an applicator keeping the hemostatic member therein and providing the hemostatic member to an affected part.

The applicator may include: a body having a space keeping the hemostatic member at a front end portion; and a handle disposed in the body to push the hemostatic member out of the body so that the hemostatic member expands in a flower shape and is provided to a hemostasis-requiring portion.

The body may have: the space keeping the hemostatic member; and a handle housing portion having a cross-sectional area smaller than the space, communicating with the space, and elongated in a longitudinal direction.

A front end of the space may be curved and an outlet that is elastically opened by a pushing force of the hemostatic member when the hemostatic member comes out of the body may be formed at the front end.

Further, a mark may be formed on an outer surface of the body so that an insertion depth of the body in a human body is recognized, and a grip may be formed at a side of the outer surface of the body.

Further, stoppers may be formed on an inner surface of the handle housing portion to prevent the handle from unexpectedly separating forward from the body.

Further, the handle may have: a body portion kept in the handle housing portion to be movable forward and rearward into and out of the handle hosing portion; a first expansion formed at the front end of the body portion, disposed in the space to be movable forward and rearward in the space, and configured to be stopped at a rear end of the space; and a second expansion formed at a rear end of the body portion to be movable forward and rearward outside the handle housing portion, and configured to be stopped at a rear end of the handle housing portion.

### Advantageous Effects

The present invention provides a hemostatic member that is inserted in an affected part and efficiently manages a hemostasis-requiring portion when the cervical tissues are examined or a surgical operation is performed on the cervix.

The present invention provides a hemostatic member that is provided in a flower shape when it is inserted into an affected part unlike the hemostatic members having a round shape in the related art. The hemostatic member is made of a hemostatic dressing and has several petals that freely close or open, depending on an affected part, so the hemostatic member can be brought in close contact with an affected part in a large contact area. Accordingly, there is an effect that the hemostatic member is useful for hemostasis.

The present invention provides a hemostatic member formed in a shape that is advantageous in hemostasis management after biopsy and conization, so the hemostatic member has excellent hemostatic ability for cervical indications. In particular, since the hemostatic member is formed in a flower shape, users of the cervical hemostatic member are provided with aesthetic effect.

Further, the hemostatic member according to the present invention is provided in a state closed in a bud shape and expands in a flower shape when it is inserted in an affected part for hemostasis. Accordingly, the bud shape enables easy insertion into an affected part and easy storage. Further, since the hemostatic member expands in a flower shape after being inserted in an affected part, it is advantageous in hemostasis management.

Further, the present invention provides a hemostatic member kit for the cervix including the hemostatic member and an applicator. The applicator is inserted into the cervix with the hemostatic member accommodated therein and then pushes out the hemostatic member so that the hemostatic member is provided to a hemostasis-requiring portion. Accordingly, the hemostatic member is easily and accurately provided to a hemostasis-requiring portion by the applicator such that the hemostatic member comes in close contact with the hemostasis-requiring portion, which is very advantageous in hemostasis management.

### Description of Drawings

FIG. 1 is a perspective view of a hemostatic member for the cervix according to an embodiment of the present invention;
FIG. 2 is a front view of the hemostatic member for the cervix according to an embodiment of the present invention;
FIGS. 3A and 3B are schematic views of a rolling member of the hemostatic member for the cervix according to an embodiment of the present invention;
FIG. 4 is a perspective view of an applicator of a hemostatic member kit for the cervix according to an embodiment of the present invention;
FIG. 5 is a rear perspective view of the applicator of a hemostatic member kit for the cervix according to an embodiment of the present invention;
FIG. 6 is a view showing the rear of the applicator of a hemostatic member kit for the cervix according to an embodiment of the present invention;
FIG. 7 is a perspective view of a hemostatic member kit for the cervix according to an embodiment of the present invention;
FIGS. 8A, 8B, 8c and 8D are schematic views showing the operation of a hemostatic member kit for the cervix according to an embodiment of the present invention;
FIGS. 9A, 9B, 9C, 9D and 9E are views showing the use state of a hemostatic member kit for the cervix according to an embodiment of the present invention; and
FIG. 10 is a schematic view of the hemostatic member for the cervix according to an embodiment of the present invention for indications.

### Mode for Invention

The present invention relates to a hemostatic member for the cervix that is inserted in an affected part to stop bleeding at a hemostasis-requiring portion in a cervical tissue examination or a surgical operation of the cervix.

In particular, the present invention relates to a hemostatic member for the cervix that has a flower shape to effectively stop bleeding for each indication at a hemostasis-requiring portion beyond the round lump shape of a hemostatic lump of the existing hemostatic member.

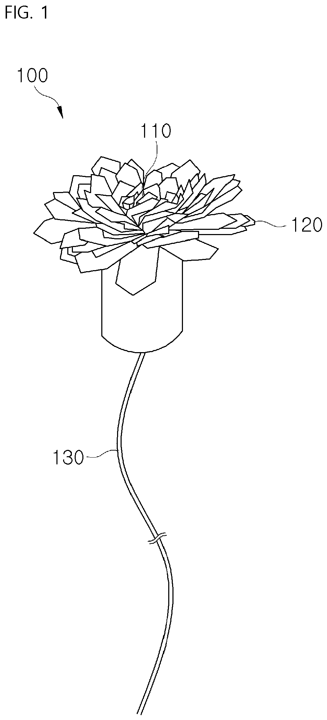

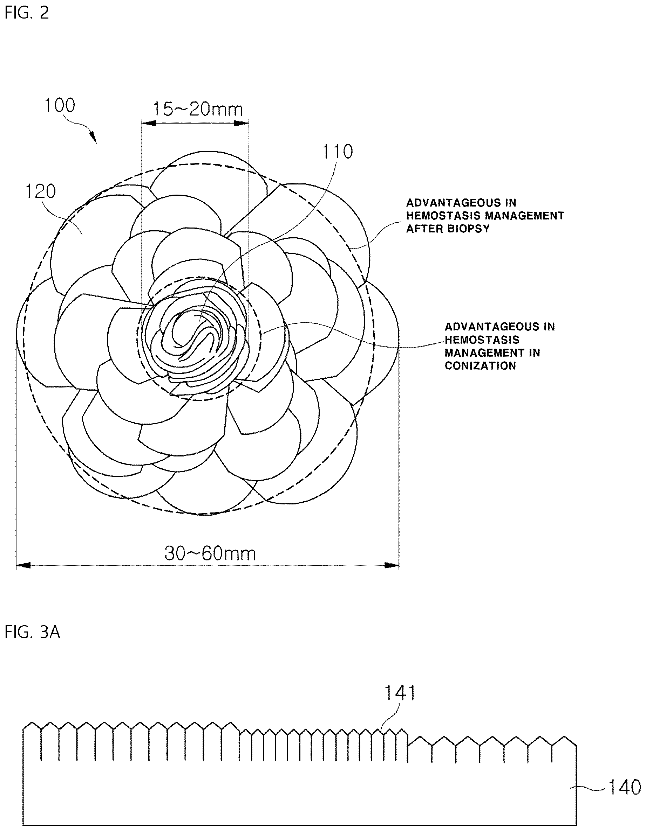

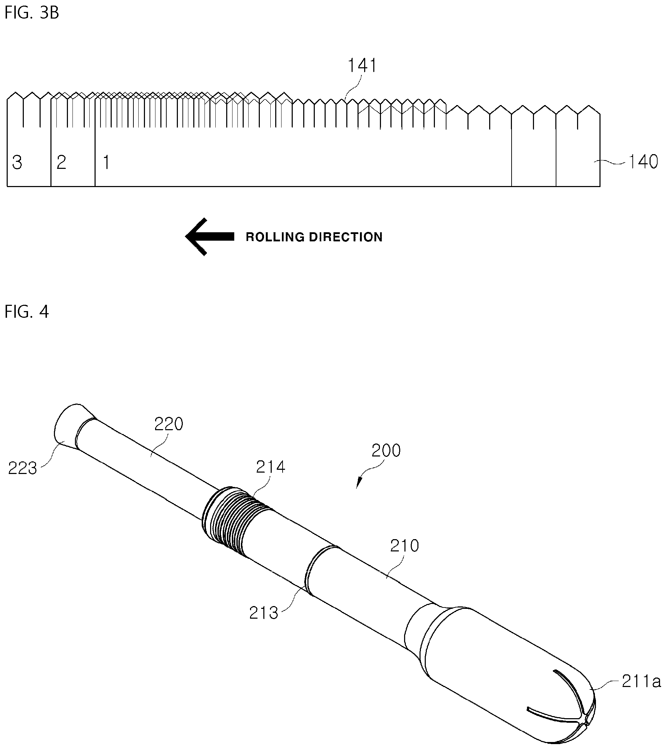

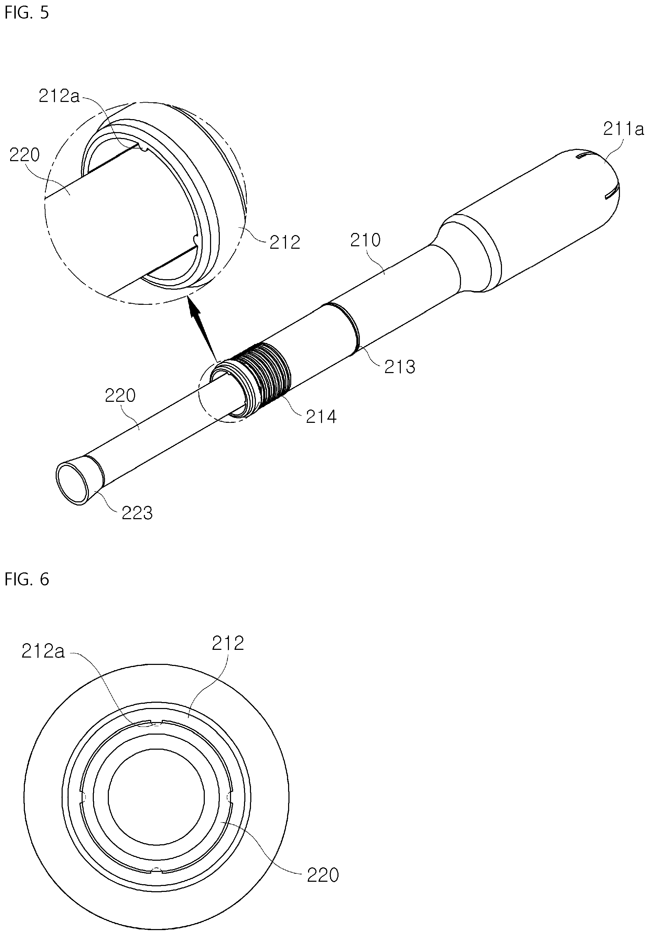

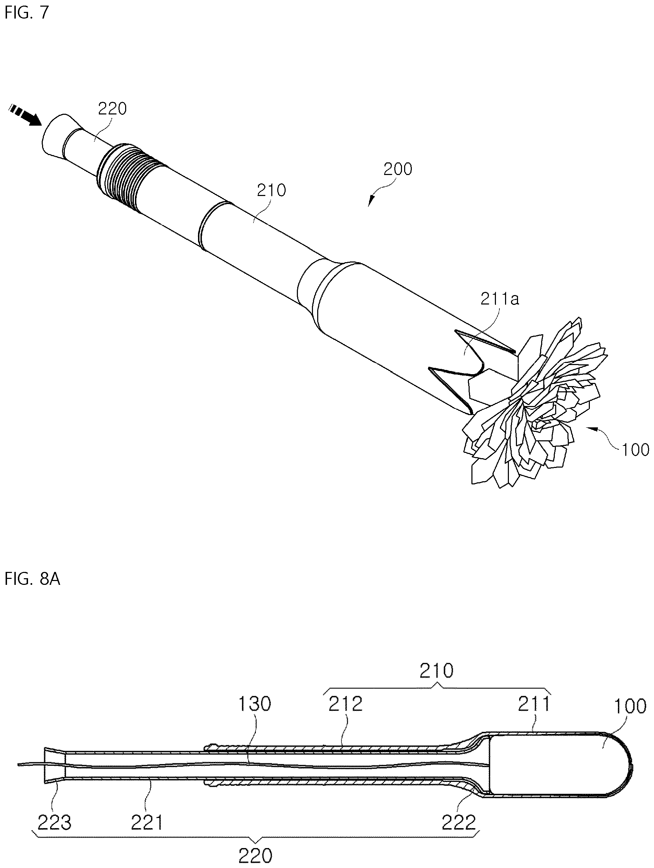

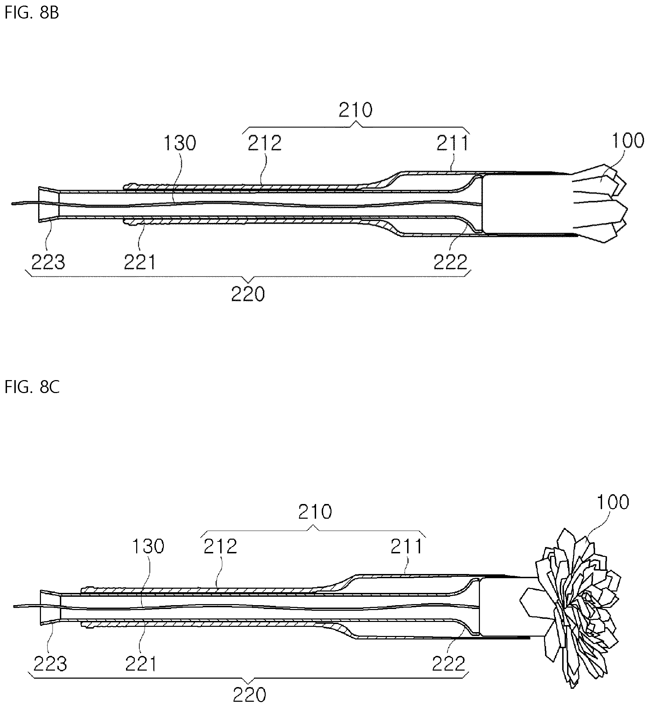

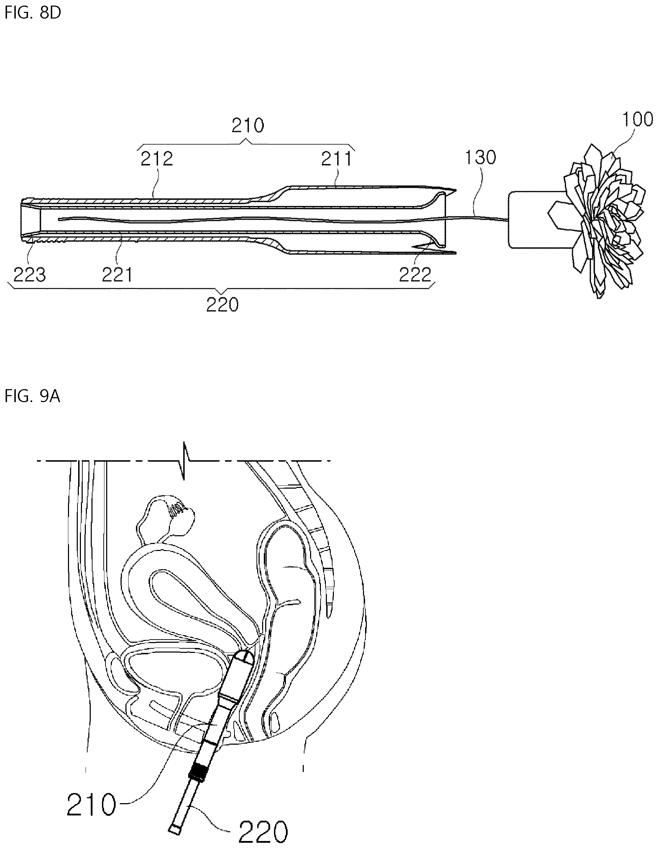

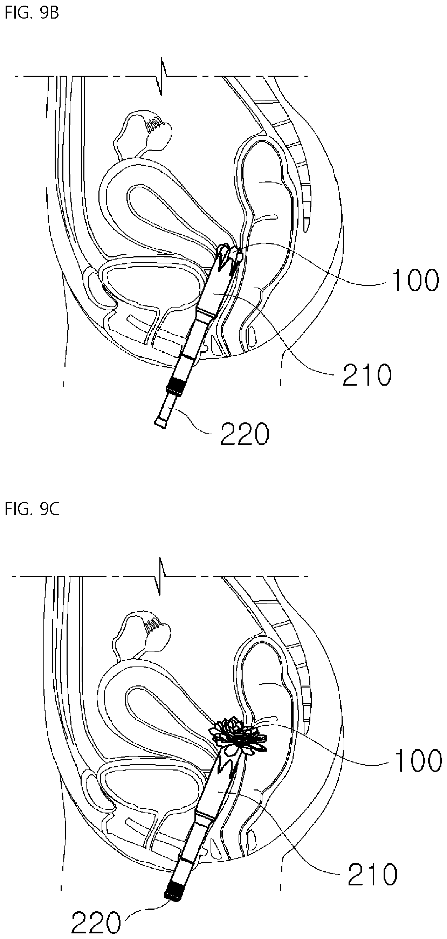

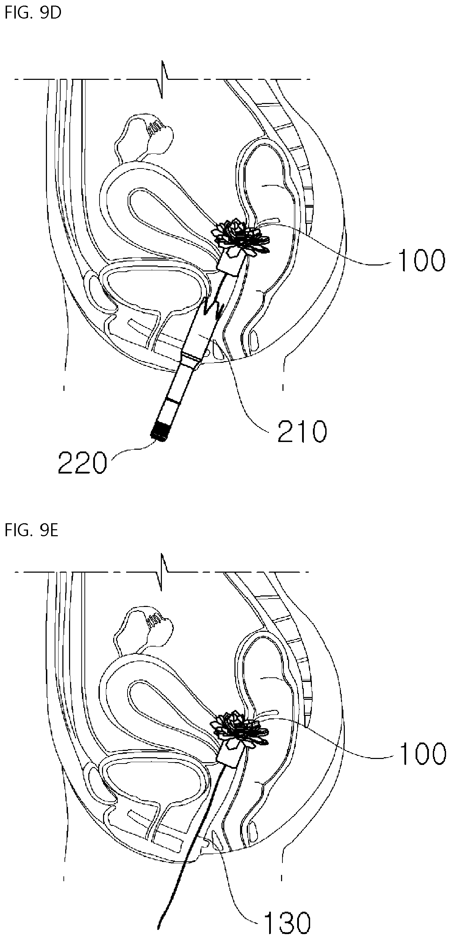

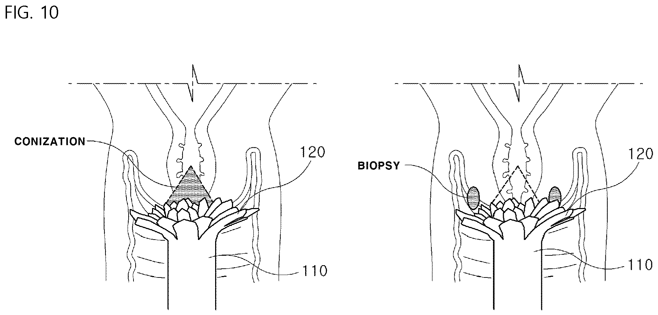

Embodiments of the present invention will be described hereafter in detail with reference to the accompanying drawings. FIG. 1 is a perspective view of a hemostatic member for the cervix according to an embodiment of the present invention, FIG. 2 is a front view of the hemostatic member for the cervix according to an embodiment of the present invention, FIGS. 3A and 3B are schematic views of a rolling member of the hemostatic member for the cervix according to an embodiment of the present invention, FIG. 4 is a perspective view of an applicator of a hemostatic member kit for the cervix according to an embodiment of the present invention, FIG. 5 is a rear perspective view of an applicator of a hemostatic member kit for the cervix according to an embodiment of the present invention, FIG. 6 is a view showing the rear of the applicator of a hemostatic member kit for the cervix according to an embodiment of the present invention, FIG. 7 is a perspective view of a hemostatic member kit for the cervix according to an embodiment of the present invention, FIGS. 8A, 8B, 8C and 8D are schematic views showing the operation of a hemostatic member kit for the cervix according to an embodiment of the present invention, FIGS. 9A, 9B, 9C, 9D and 9E are views showing the use state of a hemostatic member kit for the cervix according to an embodiment of the present invention, and FIG. 10 is a schematic view of the hemostatic member for the cervix according to an embodiment of the present invention for indications.

As shown in FIG. 1, a hemostatic member 100 according to the present invention is made of a hemostatic dressing, has a flower shape with a plurality of petal parts 120 and is characterized in that the petal parts 120 selectively close in a bud shape or open when the hemostatic member 100 is applied to an affected part.

The present invention provides a hemostatic member 100 that is provided in a flower shape when it is inserted into an affected part unlike the hemostatic members having a round shape in the related art. The hemostatic member 100 is made of a hemostatic dressing and has a plurality of petal parts 120 that freely closes or opens, depending on an affected part, so the hemostatic member 100 can be brought in close contact with an affected part in a large contact area. Accordingly, there is an effect that the hemostatic member 100 is useful for hemostasis.

In particular, round hemostatic members are not brought in close contact with the side of a spherical affected part such as the cervix, so they are insufficient to stop bleeding in the cervix.

According to the present invention, when a vaginoscope is used or the hemostatic member having a flower shape is inserted into an affected part without a vaginoscope, some of the petal parts 120 can open. Accordingly, some of the petal parts 120 close (that is, form a flower core part) and some open (that is, form a petal part), depending on the shape of an affected part.

That is, according to the hemostatic member of the present invention, only some of the petal parts 120 open or all the petal parts 120 open, depending on the shape of an affected part, to be able to quickly stop bleeding in close contact with an affected part such as the cervix.

The hemostatic member 100 for the cervix according to the present invention is made of a dressing, has a flower shape, and includes a flower core part 110 at the center and a plurality of petal parts 120 disposed around the flower core part 110.

The flower core part 110 may mean the area where the petal parts 120 do not open when the hemostatic member 100 is applied to an affected part, depending on the shape of the affected part, and the surrounding of the flower core part 110, that is, the area where the petal parts 120 open may mean the petal parts 120 contrasting with the flower core part 110.

According to an embodiment of the present invention, the petal parts 120 have dressing density lower than the flower core part 110.

The hemostatic member 100 that is provided in a flower shape when it is inserted into an affected part unlike the hemostatic members having a round shape in the related art is made of a hemostatic dressing and is composed of the flower core part 110 at the center in which the density of the dressing is relatively high, that is, the dressing is dense, and the petal parts 120 lower in density of dressing than the flower core part 110.

The diameter of the flower core part 110 may be 15∼25mm and the diameter of the hemostatic member 100 at the petal parts 120 may be 30∼60mm. That is, in the entire hemostatic member 100, the diameter of the portion where the dressing density is relatively high is 15∼25mm, that is, may be 20mm, and the diameter of the portion where the dressing density is relatively low is 30∼60mm, that is, may be 40mm. This is a size that can sufficiently cover the cervix when it is inserted in the cervix, and hemostasis is more effectively performed by the flower core part 110 having relatively high density.

The flower core part 110 may be formed with a predetermined height or may be larger in height than the petal parts 120 so that the density of the hemostatic dressing is higher than that of the flower core part 110, whereby more hemostasis can be achieved.

In particular, as shown in FIG. 2, the flower core part 110 comes in close contact with a hemostasis-requiring portion generated by conization that cuts the cervix in a cone shape and then removes dysplastic tissues or cancer tissues that are visually seen to be able to quickly stop a large amount of blood. That is, the flower core part 110 is suitable for hemostasis management after a cervical cancer operation (conization).

As described above, when the hemostatic member is applied to an affected part, the part where the petal parts 120 close at the center in correspondence to the shape of the affected part may be the flower core part 110, and the part where the petal parts 120 open around the flower core part 110 may be the petal parts 110 contrasting with the flower core part 110. Depending on affected parts, the petal parts 120 all open, so the flower core part 110 may not be discriminated in the shape, and in this case, the surface area is maximized and it may be advantageous in hemostasis management for specific affected parts.

The flower core part 110 and the petal parts 120, as described above, may be discriminated by making the shapes, height, and the dressing density of the flower core part 110 and the petal part 120 different. Further, the flower core part 110 is larger in height than the petal parts 120 and has higher dressing density to be more advantageous in hemostasis management when the hemostatic member is applied to an affected part such as the cervix and conization and biopsy are performed.

As describe above, the present invention provides a flower-shaped hemostatic member and the hemostatic member can have a large surface area due to the petal parts 120. Accordingly, the hemostatic member can be brought in close contact with a curved and amorphous affected part that is generated after biopsy and conization, so it is very suitable for hemostasis management for affected parts related to the cervix.

That is, the spherical cervical hemostatic members of the related art have difficulty in stop bleeding at the side of the cervix after biopsy, but the hemostatic member according to the present invention can easily stop bleeding at both of the center of the cervix (conization) and the side of the cervix (biopsy) due to the shape characteristic (large surface area).

When the density of the petal parts 120 (the quantity of petals) is insufficient, there may be empty portions, and in this case, it may be difficult to stop bleeding at the side of the cervix. Further, when the density of the flower core part 110 is insufficient, the dressing is not appropriately brought in close contact with a portion cut by conization, so it may be difficult to stop bleeding. Accordingly, the densities of the petal parts 120 and the flower core part 110 are adjusted for easy hemostasis management.

When the hemostatic member 100 according to the present invention is provided with the petal parts open, it may be inserted into an affected part using a vaginoscope, or when it is provided in a closed state like a bud shape, it is inserted into an affected part and then opened into a flower shape. When the hemostatic member is provided in a bud shape, it can be easily inserted into an affected part and easily kept, and after it is inserted in an affected part, it is opened in a flower shape to the advantageous in hemostasis management.

The hemostatic member 100 may further include an X-ray response substance therein. The X-ray response substance can be used to locate the hemostatic member 100 inserted in a human body.

As described above, the hemostatic member 100 having a flower shape made of a hemostatic dressing is formed in a shape that is advantageous in hemostasis management after biopsy and conization, so a hemostatic member 100 having excellent hemostatic ability for cervical indications is provided. In particular, since the hemostatic member 100 is formed in a flower shape, users of the cervical hemostatic member 100 are provided with aesthetic effect.

Further, a thread 130 is formed at the lower end of the flower core part 110 and an X-ray response material is further included in the thread 130, so it can be used for locating the hemostatic member 100 inserted in a human body. Further, it is possible to simply remove the hemostatic member 100 by pulling the end of the thread 130 when removing the hemostatic member 100 after sufficient hemostasis.

Meanwhile, the hemostatic dressing of the hemostatic member 100 according to the present invention may be made of only fiber-based nonwoven fabric, and if necessary, the dressing may be woven using a nonwoven fabric containing a functional component or may be formed by coating a nonwoven fabric with a layer made of a substance having various functional components.

The dressing is formed in a nonwoven fabric type and provided in a flower shape using any one of chitosan, cellulose (cotton, silk, or hemp), recycled cellulose (rayon or artificial silk), alginate, and casein, or by mixing chitosan in any one of cellulose (cotton, silk, or hemp), recycled cellulose (rayon or artificial silk), alginate, and casein.

That is, the dressing may be made of only fiber-based nonwoven fabric or may be made of a chitosan-based nonwoven fabric to use the useful functions of chitosan. In this case, when only chitosan is applied, gelation is generated and the material becomes flabby, so it may be difficult to maintain the strength and shape. Accordingly, cellulose (cotton, silk, or hemp), recycled cellulose (rayon or artificial silk), alginate, and casein may be mixed in chitosan.

In particular, when a dressing is fabricated by mixing rayon in chitosan, the rayon increases the absorption speed without melting even if acid treatment is applied, so it may be provided as a preferred embodiment.

Further, the dressing is mixed with a hemostatic agent and provided in a nonwoven fabric type or is provided in a nonwoven fabric type having a single coating layer or multiple coating layers made of a hemostatic agent on the surface thereof.

The hemostatic agent may be any one or a combination of two or more of carboxymethyl chitosan (CMCS), carboxymethylcellulose (CMC), chitosan succinate, sodium alginate, hyaluronic acid, collagen, gelatin, chondroitin sulfate, poly-γ-glutamic acid, pullulan, kaolin, zeolite, chondroitin sulfate, calcium chloride, calcium chloride thrombin, fibrinogen, catechol, and polyurethane foam that have excellent blood absorption ability, can coat an affected part, have excellent biocompatibility, are not noxious, and have excellent biodegradable ability.

On the other hand, as shown in FIG. 3, the hemostatic member 100 may be formed by rolling (winding) in one direction a rolling member 140 elongated in a longitudinal direction and having cuts 141 formed on a side of the rolling member 140 by the number of petals of the petal parts 120.

That is, the length of the rolling member 140 is determined in consideration of the diameter of the hemostatic member 100 and the cuts 141 are formed by the number of the petal parts 120. The cuts 141 are formed such that the height and the width increase in the rolling direction so that the sizes of the petals of the outer petal parts 120 are relatively large and the gaps thereof increase.

As shown in FIG. 3A, the rolling member 140 is elongated in the longitudinal direction in consideration of the diameter of the hemostatic member 100 and the cuts 141 corresponding to the number, shapes, sizes (widths), and lengths of the petals of the petal parts 120 are formed at the upper portion except for the portion corresponding to the height of the flower core part 110. The cuts 141 increase in length and width in the rolling direction such that the petals and the gaps of the petal parts 120 increase toward the outside from the flower core part 110, thereby providing a shape that is more advantageous in hemostasis management after biopsy.

Further, as shown in FIG. 3B, it may be possible to adjust the thickness of the hemostatic member 100 by piling up a plurality of rolling members 140, if necessary, in order to increase the size (diameter) of the hemostatic member 100. In this case, it is possible to adjust the size of the hemostatic member 100 and the density of the dressing by overlapping the plurality of rolling members 140 with stepped differences in the rolling direction.

Further, the flower core part 110 may be formed in a shape in which the lower portion decreases downward in horizontal cross-sectional area, which may be achieved by overlapping a plurality of rolling members 140 with stepped differences at the lower end or making the heights of the lower ends different.

Accordingly, since the shape characteristic in which the lower portion of the flower core part 110 narrows downward, it is possible to minimize friction due to pressure in the vagina when removing the hemostatic member after hemostasis management for the cervix, so the hemostatic member can be easily removed.

Another embodiment of the present invention provides a hemostatic member kit for the cervix. The hemostatic member kit includes: a hemostatic member 100 made of a hemostatic dressing, formed in a flower shape, having a plurality of petal parts 120 that selectively close in a bud shape or open when being applied to an affected part, and having a thread 130 at the lower end of the flower core part 120; and an applicator 200 keeping the hemostatic member 100 therein and providing the hemostatic member to a hemostasis-requiring portion.

The hemostatic member 100 according to an embodiment of the present invention is the same as the flower-shaped hemostatic member 100 described above or shown in FIGS. 1 to 3, and the applicator 200 keeps the hemostatic member 100 therein and provides the hemostatic member 100 to a hemostasis-requiring portion.

The applicator 200 is elongated in the longitudinal direction to be advantages for cervical indications and is entirely curved, that is, formed in a cylindrical shape to minimize stimulation when it is inserted into the cervix, and has a semispherical front end to be smoothly inserted into an affected part.

That is, the applicator 200 is inserted into the cervix with the hemostatic member 100 accommodated therein and then pushes out the hemostatic member 100 so that the hemostatic member 100 is provided to a hemostasis-requiring portion. The applicator 200 assists the hemostatic member 100 to be easily and accurately provided to a hemostasis-requiring portion.

The applicator 200 is made of a polyolefin-based material (LDPE, LLDPE, or HDPE) to satisfy properties for enabling the hemostatic member 100 to be smoothly pushed out of the applicator 200. Further, the material prevents damage to surrounding tissues when the applicator 200 is inserted into a human body and has a biologically secured stability.

As shown in FIGS. 4 to 8, the applicator 200 includes: a body 210 having a space 211 for keeping the hemostatic member 100 at the front end portion; and a handle 220 disposed in the body 210 to push the hemostatic member 100 out of the body 210 so that the hemostatic member 100 expands in a flower shape and is provided to a hemostasis-requiring portion.

That is, the hemostatic member 100 according to the present invention is kept in the space 211 of the body 210, and in this state, the hemostatic member 100 is closed in a bud shape. Thereafter, when a user pushes the hemostatic member 100 out of the body 210 using the handle 220 kept in the body 210, all the petal parts 120 open and expand in a flower shape while the hemostatic member 100 comes out of the body 210, whereby the flower-shaped hemostatic member 100 is provided.

Further, the body 210 has the space 211 for keeping the hemostatic member 100, and a handle housing portion 212 having a cross-sectional area smaller than the space 211, communicating with the space 211, and elongated in the longitudinal direction.

The front end of the space 211 is curved and an outlet 211a that is elastically opened by the pushing force of the hemostatic member 100 when the hemostatic member 100 comes out of the body 210 is formed at the front end.

The outlet 211a is closed when the applicator 200 is inserted into a human body, and the front end of the space 211 of the body 210 is curved to be easily inserted into a human body without stimulating the surrounding tissues. Further, after the applicator 200 is inserted in an affected part, the hemostatic member 100 comes out and the outlet 211a is split and opened by the pushing force, whereby the petal parts 120 expand and the flower-shaped hemostatic member 100 is achieved.

A mark 213 is formed on the outer surface of the body 210 so that the insertion depth of the body 210 in a human body can be recognized, and an embossed grip 214 is formed at a side of the outer surface of the body 210 so that a user conveniently insert or remove the applicator into and out of a human body.

Further, as shown in FIGS. 5 and 6, stoppers 212a are formed on the inner surface of the handle housing portion 212 to prevent the handle 220 from unexpectedly separating forward from the body 210. That is, the stoppers are provided to prevent the hemostatic member 100 from separating forward from the body 210 through the handle housing portion 212 in the movement direction of the handle when a user pushes the hemostatic member 100 out of the body 210 using the handle 220.

The handle 220 has: a body portion 221 kept in the handle housing portion 212 to be movable forward and rearward into and out of the handle hosing portion 212; a first expansion 222 formed at the front end of the body portion 221, disposed in the space 211 to be movable forward and rearward in the space 211, and configured to be stopped at the rear end of the space 211p and a second expansion 223 formed at the rear end of the body portion 221 to be movable forward and rearward outside the handle housing portion 212, and configured to be stopped at the rear end of the handle housing portion 212.

That is, the handle 220, fundamentally, can move forward and rearward in the space 211 of the body 210 and in the handle housing portion 212 and is stopped at the rear end of the space 211 by the first expansion, so the handle 220 is not separated rearward from the body 210. Further, the handle 220 is not separated forward from the body 210 by the second expansion 223. Accordingly, the handle 220 can freely move forward and rearward in the body 210, but is not separated out of the body 210.

The stoppers 212a assist the handle 220 not to unexpectedly separate forward from the body 210.

FIG. 7 shows the state in which the hemostatic member 100 kept in the applicator 200 has opened the outlet 211a while being pushed forward by the handle at the rear portion, so the hemostatic member 100 has come out of the body 210 through the open outlet 211a.

FIG. 8A shows the state in which the hemostatic member 100 according to the present invention is kept in the space 211 of the body 210 and the handle 220 is kept in the body 210 behind the hemostatic member 100 with the first expansion 222 of the handle 220 and the rear of the hemostatic member 100 in contact with each other. In this state, the outlet 211a is closed and the front end of the body 210, that is, the front end of the space 211 is maintained in a curved shape.

Referring to FIGS. 8B and 8C, as the handle 220 is pushed forward, the hemostatic member 100 kept in the space 211 comes out of the body 210, and in this process, the outlet 211a of the space 211 is elastically opened by the pushing force of the hemostatic member 100 and the hemostatic member 100 comes out of the body 210.

Referring to FIG. 8D, as the handle 220 is further pushed forward, the hemostatic member 100 kept in the space 211 fully comes out of the body 210, and in this process, the petal parts 120 fully open, whereby the hemostatic member 100 expands in a flower shape and is provided to a hemostasis-requiring portion.

Thereafter, the handle 220 and the body 210 are pulled rearward and removed out of a human body, so only the hemostatic member 100 remains in the hemostasis-requiring portion.

FIG. 9 shows a use state of the hemostatic member kit for the cervix according to the present invention. The applicator 200 keeping the hemostatic member 100 is inserted into an affected part (FIG. 9A), the hemostatic member 100 is pushed to come out of the body 210 by the handle 220 (FIG. 9B), and then after the hemostatic member 100 is provided to a hemostasis-requiring portion, the handle 220 and the body 210 are pulled out of the human body (FIG. 9D) such that only the hemostatic member 100 remains in the hemostasis-requiring portion (FIG. 9E). The thread 130 of the hemostatic member 100 is used to conveniently remove the hemostatic member 100 by being pulled by a patient or a doctor after hemostasis is finished.

FIG. 10 is a schematic view of the hemostatic member 100 for the cervix according to an embodiment of the present invention for indications.

As described above, the flower core part 110 of the hemostatic member 100 has high dressing density and a diameter of about 20mm, so it is advantageous in hemostasis management in conization that is a cervical operation. The petal parts 120 of the hemostatic member 100 are wide and have a size that can cover the cervix, so it can be usefully applied for hemostasis management after taking uterine tissues.

## Claims

1. A hemostatic member for a cervix, wherein the hemostatic member is made of a hemostatic dressing in a flower shape, including a plurality of petal parts, and
the petal parts selectively close in a bud shape or open when applied to an affected part.

2. The hemostatic member for a cervix of claim 1, wherein only some of the petal parts open or all the petal parts open, depending on a shape of an affected part.

3. The hemostatic member for a cervix of claim 1, comprising:
a flower core part formed at a center; and
the petal parts formed around the flower core part.

4. The hemostatic member for a cervix of claim 3, wherein the petal parts have lower dressing density than the flower core part.

5. The hemostatic member for a cervix of claim 3, wherein the flower core part is larger in height than the petal parts.

6. The hemostatic member for a cervix of claim 3, wherein a thread is formed at a lower end of the flower core part.

7. The hemostatic member for a cervix of claim 3, wherein the thread further includes an X-ray response substance.

8. The hemostatic member for a cervix of claim 1, wherein the dressing is any one of chitosan, cellulose (cotton, silk, or hemp), recycled cellulose (rayon or artificial silk), alginate, and casein, or is provided by mixing chitosan in any one of cellulose (cotton, silk, or hemp), recycled cellulose (rayon or artificial silk), alginate, and casein.

9. The hemostatic member for a cervix of claim 8, wherein the dressing is provided in a nonwoven fabric type mixed with a hemostatic agent or is provided in a nonwoven fabric type having a single coating layer or multiple coating layers made of a hemostatic agent on a surface thereof.

10. The hemostatic member for a cervix of claim 9, wherein the hemostatic agent is any one or a combination of two or more of carboxymethyl chitosan (CMCS), carboxymethylcellulose (CMC), chitosan succinate, sodium alginate, hyaluronic acid, collagen, gelatin, chondroitin sulfate, poly-γ-glutamic acid, pullulan, kaolin, zeolite, chondroitin sulfate, calcium chloride, calcium chloride thrombin, fibrinogen, catechol, and polyurethane foam.

11. The hemostatic member for a cervix of claim 1, wherein the hemostatic includes an X-ray response substance.

12. The hemostatic member for a cervix of claim 1, wherein a diameter of the flower core part is 15 ∼ 25mm and a diameter of the hemostatic member at the petal parts is 30 ∼ 60mm.

13. The hemostatic member for a cervix of claim 1, wherein the hemostatic member is formed by rolling in one direction a rolling member elongated in a longitudinal direction and having cuts formed on a side of the rolling member by the number of petals of the petal parts.

14. The hemostatic member for a cervix of claim 13, wherein the cuts are formed such that a height and a width increase in a rolling direction so that sizes of the outer petal parts are relatively large and gaps thereof increase.

15. The hemostatic member for a cervix of claim 13, wherein a size (diameter) of the hemostatic member is adjusted by piling up a plurality of rolling members or the size (diameter) and density of the hemostatic member are adjusted by overlapping a plurality of rolling members with stepped difference in a rolling direction.

16. The hemostatic member for a cervix of claim 3, wherein the flower core part is formed in a shape in which a lower portion decreases downward in horizontal cross-sectional area.

17. A hemostatic member kit for a cervix, comprising:
a hemostatic member made of a dressing in a flower shape, including a plurality of petal parts that selectively close in a bud shape or open when applied to an affected part, and having a thread at a lower end of the flower core part; and
an applicator keeping the hemostatic member therein and providing the hemostatic member to an affected part.

18. The hemostatic member kit for a cervix of claim 17, wherein the applicator comprises:
a body having a space keeping the hemostatic member at a front end portion; and
a handle disposed in the body to push the hemostatic member out of the body so that the hemostatic member expands in a flower shape and is provided to a hemostasis-requiring portion.

19. The hemostatic member kit for a cervix of claim 18, wherein the body comprises:
the space keeping the hemostatic member; and
a handle housing portion having a cross-sectional area smaller than the space, communicating with the space, and elongated in a longitudinal direction.

20. The hemostatic member kit for a cervix of claim 19, wherein a front end of the space is curved and an outlet that is elastically opened by a pushing force of the hemostatic member when the hemostatic member comes out of the body is formed at the front end.

21. The hemostatic member kit for a cervix of claim 19, wherein stoppers are formed on an inner surface of the handle housing portion to prevent the handle from unexpectedly separating forward from the body.

22. The hemostatic member kit for a cervix of claim 19, wherein the handle comprises:
a body portion kept in the handle housing portion to be movable forward and rearward into and out of the handle hosing portion;
a first expansion formed at the front end of the body portion, disposed in the space to be movable forward and rearward in the space, and configured to be stopped at a rear end of the space; and
a second expansion formed at a rear end of the body portion to be movable forward and rearward outside the handle housing portion, and configured to be stopped at a rear end of the handle housing portion.

23. The hemostatic member kit for a cervix of claim 18, wherein a mark is formed on an outer surface of the body so that an insertion depth of the body in a human body is recognized.

24. The hemostatic member kit for a cervix of claim 18, wherein a grip is formed at a side of the outer surface of the body.
